(19)

Europäisches
Patentamt
European
Patent Office
Office européen
des brevets

(11) **EP 2 621 336 B1**

(12) **EUROPEAN PATENT SPECIFICATION**

(45) Date of publication and mention
of the grant of the patent:
**22.07.2015   Bulletin 2015/30**

(21) Application number: **11773872.4**

(22) Date of filing: **23.09.2011**

(51) Int Cl.:
***A61B 5/087*** (2006.01)          ***A61B 7/00*** (2006.01)

(86) International application number:
**PCT/IB2011/054201**

(87) International publication number:
**WO 2012/042453 (05.04.2012 Gazette 2012/14)**

(54) **APPARATUS AND METHOD FOR DIAGNOSING OBSTRUCTIVE SLEEP APNEA**

VORRICHTUNG UND VERFAHREN ZUR DIAGNOSE VON OBSTRUKTIVER SCHLAFAPNOE

APPAREIL ET PROCÉDÉ POUR DIAGNOSTIQUER UNE APNÉE OBSTRUCTIVE DU SOMMEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **01.10.2010  EP 10185347**

(43) Date of publication of application:
**07.08.2013   Bulletin 2013/32**

(73) Proprietors:
• **Koninklijke Philips N.V.**
 **5656 AE Eindhoven (NL)**
 Designated Contracting States:
 **AL AT BE BG CH CY CZ DK EE ES FI FR GB GR
 HR HU IE IS IT LI LT LU LV MC MK MT NL NO PL
 PT RO RS SE SI SK SM TR**
• **Philips Intellectual Property & Standards GmbH
 20099 Hamburg (DE)**
 Designated Contracting States:
 **DE**

(72) Inventors:
• **MUESCH, Guido**
 **NL-5656 AE Eindhoven (NL)**
• **PINTER, Robert**
 **NL-5656 AE Eindhoven (NL)**
• **GOURMELON, Lena**
 **NL-5656 AE Eindhoven (NL)**

(74) Representative: **Steffen, Thomas
 Philips
 Intellectual Property & Standards
 P.O. Box 220
 5600 AE Eindhoven (NL)**

(56) References cited:
**US-A1- 2007 093 724     US-B1- 7 740 591
US-B2- 6 942 626**

**Description**

TECHNICAL FIELD OF THE INVENTION

The invention relates to an apparatus and method for collecting information, and in particular to an apparatus and method for collecting information from a patient that is awake and that can be used in diagnosing obstructive sleep apnea in the patient.

BACKGROUND TO THE INVENTION

Obstructive sleep apnea (OSA) is a condition in which a subject experiences a decrease or complete stop in airflow while asleep, despite the subject continuing to try to breathe. These events occur when the muscles relax during sleep, causing soft tissue in the back of the throat to collapse and block the upper airway. This leads to partial reductions (known as hypopneas) and complete pauses (known as apneas) in breathing. An apnea event is defined as a cessation of airflow for at least 10 seconds during sleep. Hypopnea is defined as an abnormal respiratory event lasting at least 10 seconds with at least a 30 percent reduction in thoracoabdominal movement or airflow as compared to a baseline, with at least a 4 percent oxygen desaturation. Most apnea events last between 10 and 30 seconds, but some may persist for one minute or longer. This can lead to abrupt reductions in blood oxygen saturation, with oxygen levels falling as much as 40 percent or more in severe cases.

These apnea events cause the subject to wake briefly which restores normal breathing. As these apneas can occur tens or hundreds of times per night, the disruption caused results in the subject being excessively tired during the day.

A common measurement of sleep apnea is the apnea-hypopnea index (AHI). This is a number that represents the combined number of apneas and hypopneas that occur per hour of sleep. The following classification is frequently used:

| | |
|---|---|
| AHI < 5: | No OSA / Healthy |
| 5 < AHI <15: | Mild OSA |
| 15 < AHI < 30: | Moderate OSA |
| 30 < AHI | Severe OSA |

Generally, obstructive sleep apnea (OSA) is diagnosed in a sleep laboratory. However, most patients suffering from obstructive sleep apnea are never properly diagnosed since primary care physicians frequently deal with the symptoms of daytime fatigue and poor sleep by prescribing sleeping pills or similar medication. Physicians can be hesitant to send patients to a sleep laboratory immediately because of the high cost involved and the long waiting times. Usually patients are only sent when all other treatment attempts have failed and the patient keeps on complaining about bad sleep and daytime sleepiness. However, once a patient with suspected OSA is sent to a sleep laboratory, OSA is confirmed in around 85% of the cases.

OSA is diagnosed in a sleep laboratory with the help of "polysomnography" which is performed over one or more nights while the patient is asleep. Polysomnography can involve the use of an electroencephalogram (EEG), an electrocardiogram (ECG), an electroculogram (EOG), an electromyogram (EMG) and/or respiratory chest bands and the measurement of nasal airflow, blood oxygen levels and/or other physiological parameters. As a large number of sensors and devices are required for polysomnography, this procedure is not very comfortable or convenient for the patient.

Generally, the events of apnea and hypopnea in the polysomnography data are identified by a physician manually inspecting short intervals (roughly 30 seconds) of data, and individually rating the relevance of those intervals. Apnea events are characterized by the airflow through the patient's nasal passage stopping (or nearly stopping) while the thoracic and abdominal breathing movement continues. The number of identified events are counted and the average number of events per hour is used as an indicator of whether the patient has OSA and, if so, its severity.

However, a substantial amount of effort is required to scan the data covering a whole night in order to detect and count all apnea and hypopnea events, and to determine the AHI value for a patient.

Alternative techniques for diagnosing OSA have been proposed that involve the investigation of the snoring sounds of a patient. One such technique is described in "Investigation of Obstructive Sleep Apnea Using Nonlinear Mode Interactions in Nonstationary Snore Signals" by Ng et al., Annals of Biomedical Engineering, Vol. 37, No. 9, September 2009, pp. 1796-1806. However, this technique again requires the patient to attend a sleep laboratory and to be monitored while they are sleeping.

Therefore, there is a need for a more efficient method and apparatus for OSA screening, and that can be used while the patient is awake. Such a method and apparatus would allow more patients with suspected or possible OSA to be tested and would increase the number of patients with OSA that receive appropriate treatment for their condition.

One such method and apparatus that can be used while the patient is awake is disclosed in US 6,942,626. However, applying this method to a larger data set could not provide reliable results.

In addition, the 'snore analysis' technique described above cannot be applied to data obtained from a patient that is awake since muscle tension persists in the upper airway of the awake patient, and this means that the signal processing techniques, which have been developed and optimized for signals obtained from sleeping patients, are not able to provide useful results.

US2007/093724 discloses an automatic apnea/hypopnea detection device. The device includes a respirometer and an automatic apnea/hypopnea analyzer. The automatic apnea/hypopnea analyzer obtains power spectra in the breathing frequency band. US6,942,626 discloses an apparatus for identifying sleep disordered breathing while the patient is awake. The device includes collection of raw data which is normalized, then conditioned and transformed for comparison against a threshold, enabling identification of the presence and severity of sleep disordered breathing.

## SUMMARY OF THE INVENTION

The invention provides a fast and comfortable OSA testing apparatus that is to be used while the patient is awake and a method of collecting information relating to obstructive sleep apnea while the patient is awake. The OSA test is targeted at detecting abnormalities of the upper airway in patients that have OSA and that influence the airflow during normal breathing while the person is awake.

Therefore, according to a first aspect of the invention, there is provided an apparatus for determining one or more parameters for use in diagnosing obstructive sleep apnea in a patient while the patient is awake, the apparatus being according to claim 1 and comprising a processor configured to receive signals representing measurements of a patient's breathing obtained during a plurality of breathing cycles by the patient, convert the signals into the frequency domain and determine a value for at least one parameter based on an analysis of the frequency-domain converted signals in one or more frequency bands covering frequencies below 100 Hz and as further defined in claim 1.

In one embodiment, the processor is configured to output the value for the at least one parameter to an operator of the apparatus.

In another embodiment, the processor is configured to determine whether the patient is likely to have obstructive sleep apnea based on the value of the at least one parameter and to output an indication of the likely presence or absence of obstructive sleep apnea in the patient to an operator of the apparatus.

Preferably, the processor is configured to determine whether the patient is likely to have obstructive sleep apnea based on a combination of values for a plurality of parameters.

The processor can be configured to determine a value for a first parameter by comparing the signals in a first frequency band covering frequencies below 100 Hz during exhalation to the signals in a second frequency band covering frequencies below 100 Hz during exhalation. Preferably, the first frequency band is 20-50 Hz, or, more preferably, 25-45 Hz, or, even more preferably, 30-40 Hz. Preferably, the second frequency band is 12-30 Hz, or, more preferably, 15-25 Hz, or, even more preferably, 18-22 Hz. Thus, in a preferred embodiment, the value for the first parameter is determined by comparing the signals during exhalation in the frequency band 30-40 Hz and the signals during exhalation in the frequency band 18-22 Hz.

In addition or alternatively to the first parameter described above, the processor can be configured to determine a value for a second parameter by comparing the signals in a third frequency band covering frequencies below 100 Hz during inhalation to the signals in the third frequency band during exhalation. Preferably the third frequency band is 0-20 Hz, or, more preferably, 0-15 Hz, or, even more preferably, 0-10 Hz.

In addition or alternatively to the first and second parameters described above, the processor can be configured to determine a value for a third parameter by comparing the signals in a fourth frequency band covering frequencies below 100 Hz during an inhalation or exhalation to a noise level above a threshold frequency during the inhalation or exhalation. Preferably, the fourth frequency band is 0-100 Hz and the threshold frequency for the noise level is 100 Hz or above, for example 200 Hz or 2000 Hz.

In a further embodiment, the processor is configured to determine a value for at least one further parameter based on a time domain analysis of the signals.

In yet another further embodiment, the processor is configured to determine a value for at least one further parameter, where the further parameter or parameters are selected from (i) the average length of a breathing cycle; and (ii) the ratio of the length of the inhalation to the length of the exhalation.

Preferably, the processor is configured to receive signals indicative of the rate of air flow during the plurality of breathing cycles by the patient while the patient is awake. In one embodiment, the apparatus further comprises an air flow measuring device for measuring the flow rate of air over time during the plurality of breathing cycles by the patient while the patient is awake and for generating the signals indicative of the rate of air flow during the breathing cycles.

In an alternative embodiment, the processor is configured to receive signals indicative of the sound of the patient's breathing during the plurality of breathing cycles by the patient while the patient is awake. In a further embodiment, the apparatus further comprises a sound measuring device for measuring the sound of the air flow over time during the plurality of breathing cycles by the patient while the patient is awake and for generating the signals indicative of the sound of the patient's breathing.

In one embodiment, the processor is configured to convert the signals into the frequency domain by performing a respective Fast Fourier Transform, FFT, on the signals in each inhalation and exhalation part of the breathing cycle.

However, in a preferred embodiment, the processor is configured to convert the signals into the frequency domain by identifying the peak air flow during each inhalation and exhalation part of the breathing cycle and performing a Fast Fourier Transform, FFT, on the signals around the peak flows in each inhalation and exhalation part of the breathing cycle.

In preferred embodiments, the at least one parameter comprises:

(i) a comparison of the signals in a first frequency band covering frequencies below 100 Hz during exhalation to the signals in a second frequency band covering frequencies below 100 Hz during exhalation;
(ii) a comparison of the signals in a third frequency band covering frequencies below 100 Hz during inhalation to the signals in the third frequency band during exhalation; and/or
(iii) a comparison of the signals in a fourth frequency band covering frequencies below 100 Hz during an inhalation or exhalation to a noise level above a frequency threshold during the inhalation or exhalation.

In these preferred embodiments, the first frequency band is preferably 20-50 Hz, or, more preferably, 25-45 Hz, or, even more preferably, 30-40 Hz, the second frequency band is preferably 12-30 Hz, or, more preferably, 15-25 Hz, or, even more preferably, 18-22 Hz, the third frequency band is preferably 0-20 Hz, or, more preferably, 0-15 Hz, or, even more preferably, 0-10 Hz, the fourth frequency band is preferably 0-100Hz and the frequency threshold is 100 Hz or above, for example 200 Hz or 2000 Hz.

Further embodiments can comprise the step of determining a value for at least one further parameter based on a time domain analysis of the signals. In these further embodiments, the at least one further parameter can comprise (i) the average length of a breathing cycle; and (ii) the ratio of the length of the inhalation to the length of the exhalation.

In some embodiments, the step of converting comprises performing a respective Fast Fourier Transform, FFT, on the signals in each inhalation and exhalation part of the breathing cycle. However, in alternative embodiments, the step of converting comprises identifying the peak air flow during each inhalation and exhalation part of the breathing cycle and performing a Fast Fourier Transform, FFT, on the signals around the peak flows in each inhalation and exhalation part of the breathing cycle.

Thus, the invention provides an apparatus that can perform a fast, cost-effective and comfortable test on a patient that is awake in order to diagnose OSA and a method that provides information to a physician that allows them to determine if the patient has OSA.

BRIEF DESCRIPTION OF THE DRAWINGS

Exemplary embodiments of the invention will be described in detail below with reference to the following drawings, in which:

Fig. 1 is a block diagram of an apparatus according to the invention;
Fig. 2 is a flow chart showing the functional steps in a method according to an embodiment of the invention;
Figs. 3(a) and (b) are graphs illustrating the filtering process performed in a preprocessing step;
Fig. 4 illustrates the application of a sliding window FFT to only a part of each inhalation or exhalation segment around the peak air flow; and
Figs. 5(a) and (b) illustrate a typical frequency spectrum for a healthy patient and a patient with obstructive sleep apnea respectively.

DETAILED DESCRIPTION OF THE PREFERRED EMBODIMENTS

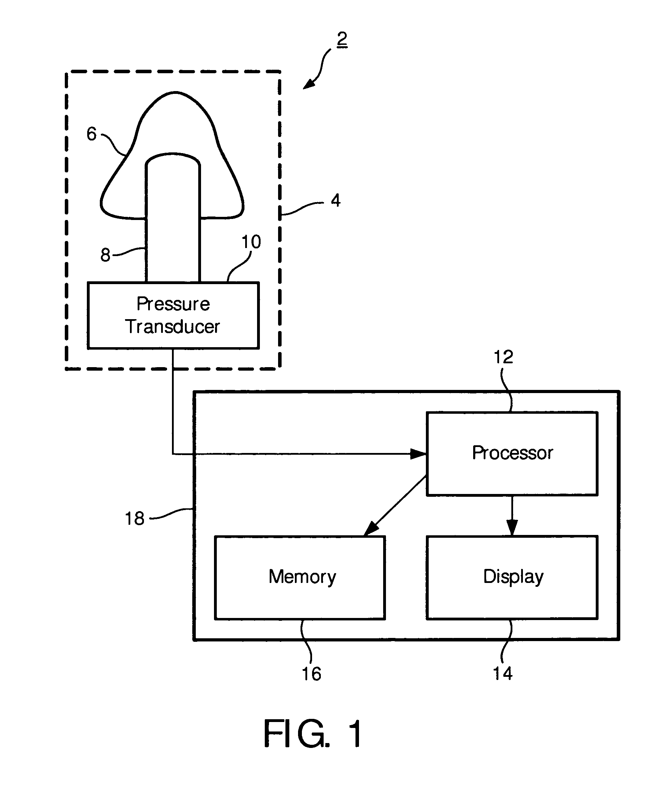

Figure 1 shows an exemplary apparatus 2 according to the invention that can be used in the detection of obstructive sleep apnea in a patient based on data that is collected from the patient while they are awake.

In a preferred embodiment, the apparatus 2 comprises an air flow measuring device 4, such as a pneumotachograph, for providing measurements of the flow of air during inhalations and exhalations by a patient. As known, a pneumotachograph 4 comprises a nasal mask, facial mask or mouthpiece 6 that can be worn by the patient, a pneumotachometer 8 that is connected to the nasal mask, facial mask or mouthpiece 6, that measures the flow of air being inhaled and exhaled by the patient through the nasal mask, facial mask or mouthpiece 6 and provides an output in terms of a differential pressure, and a pressure transducer 10 that is connected to the pneumotachometer 8 and that converts the differential pressure output into an electrical signal, preferably digital samples.

The electrical signal is provided from the pressure transducer 10 in the pneumotachograph 4 to a processor 12 where it is processed to determine information that can be used by a physician to determine whether the patient has a sleep-related breathing disorder, such as obstructive sleep apnea (OSA). The processor 12 is connected to a display 14 that provides a visual indication of the result of the processing (such as the information to be used by the physician in diagnosing the patient, and/or, in some implementations of the invention, an indication of whether the patient has OSA or other breathing disorder). The processor 12 is also connected to a memory 16 that can store the electrical signals output from the pneumotachograph 4 prior to processing by the processor 12, as well as any result or results of the processing performed by the processor 12 on the electrical signals.

In this illustrated embodiment, the processor 12, display 14 and memory 16 are contained in a processing unit 18 that forms a separate unit to the pneumotachograph 4. In this case, the electrical signals from the pneumotachograph 4 can be provided to the processor 12 in the processing unit 18 via a connecting wire, wirelessly using WiFi, Bluetooth, etc., or by any other suitable means. However, in alternative implementations, the pneumotachograph 4 and processing unit 18 can be provided within a single housing. In either case, the apparatus 2 is preferably implemented as a lightweight device that can be easily held or worn by the patient during a testing procedure without causing the patient undue discomfort.

Although not shown in Figure 1, it will be appreciated that the apparatus 2 (and in particular the processing unit 18) may include additional components, such as a user interface for allowing a user of the apparatus 2 to input commands and/or patient-specific data to the processor 12 and/or an internal power supply such as a battery if the apparatus 2 is to be operated independently of an external power supply.

In alternative embodiments, the pneumotachograph 4 can be replaced by an alternative means that can provide measurements of air flow, such as a nasal cannula.

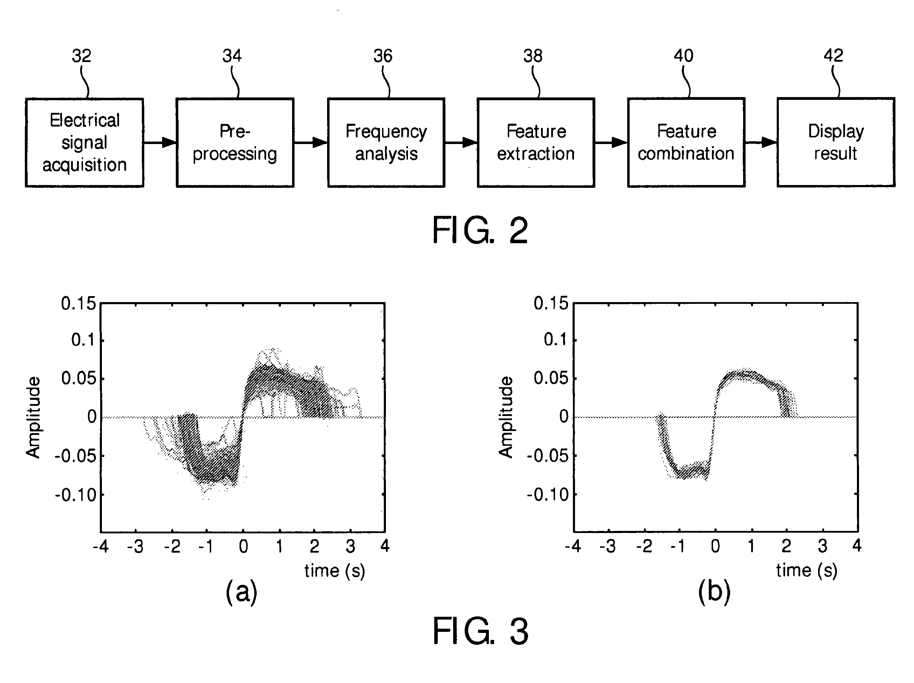

Figure 2 is a functional diagram illustrating the operations performed by or in the apparatus 2 according to the invention. In a first step 32, electrical signals representing the air flow to and from the patient's lungs during breathing while the patient is awake are acquired from the pneumotachograph 4. The electrical signals preferably comprise digital samples representing the magnitude (i.e. rate) of the air flow at respective sampling instants. As suggested above, the first step 32 is performed while the patient is awake.

The air flow rate samples are passed to the processor 12 where they are processed to provide information relating to the breathing condition of the patient. In some embodiments this information is presented to a physician to assist the physician in diagnosing obstructive sleep apnea. In other embodiments, the processor can further process the information to provide an indication of whether the patient has obstructive sleep apnea, which can be output by the apparatus 2 to an operator (such as a physician), for example using the display 14.

It has been found that the raw sample data can contain artifacts (for example see Figure 3(a) discussed below), which can affect the quality of the analysis performed in subsequent processing steps. Therefore, it is desirable to provide a step that assesses the quality of the raw sample data and selects a subset of the data for one or more breathing cycles that are to be used in the subsequent processing steps. Thus, the first processing step performed by the processor 12 is a pre-processing step (step 34 in Figure 2) in which the raw sample data is processed to identify N breathing cycles (with a single breathing cycle comprising a consecutive inhalation and exhalation) that are to be used in subsequent processing steps. Preferably, the N breathing cycles selected are those breathing cycles that best fit a mean breathing cycle for the patient. In preferred embodiments N is 12, although N can take any positive integer value.

The selection of the N breathing cycles is preferably performed as follows. Firstly, the raw sample data is separated into individual breathing cycles, and preferably individual inhalation and exhalation segments. The transition points between each inhalation and exhalation (i.e. where the patient starts to exhale after inhaling and exhaling after inhaling) can be easily identified from the zero-crossings in the sample data.

Next, the breathing cycles or individual inhalation and exhalation segments are filtered using one or more criteria, for example, a minimum length, the deviation from a mean length (in total and also separately for inhalation and exhalation segments) and deviation from a mean shape. The N cycles or segments best meeting the required criteria are then selected for further analysis by the processor 12.

Figures 3(a) and 3(b) illustrate the filtering process performed in the preprocessing step 34. Figure 3(a) is a graph plotting the sample data obtained from a patient, with the data split into individual breathing cycles. The transition between inhalation and exhalation in each breathing cycle is plotted at the origin of the graph. The samples with a negative amplitude represent air flowing into the patient's lungs (i.e. during inhalation), and the samples with a positive amplitude represent air flow out of the patient's lungs (i.e. during exhalation). Thus, it can be seen that the air flow in many of the breathing cycles follows a generally regular pattern, but there are a number of breathing cycles in which the air flow varies considerably from the regular pattern (i.e. they contain artifacts). The filtering step described above results in the selection of N=12 breathing cycles fitting a mean breathing cycle for the patient, as shown in Figure 3(b).

In one embodiment of the invention, in order to reduce the amount of time that a patient has to be attached to the testing apparatus 2, the processor 12 can perform the pre-processing step while the data is being collected, and can provide an indication to the patient or other user of the apparatus 2 that the test can be stopped once the data for N breathing cycles has been collected.

After the preprocessing step, the processor 12 performs a frequency analysis step 36 in which the sample data is converted into the frequency domain and a mean frequency spectrum is calculated. In particular, a sliding window Fast Fourier Transform (FFT) is applied to each individual breathing cycle to give a frequency spectrum.

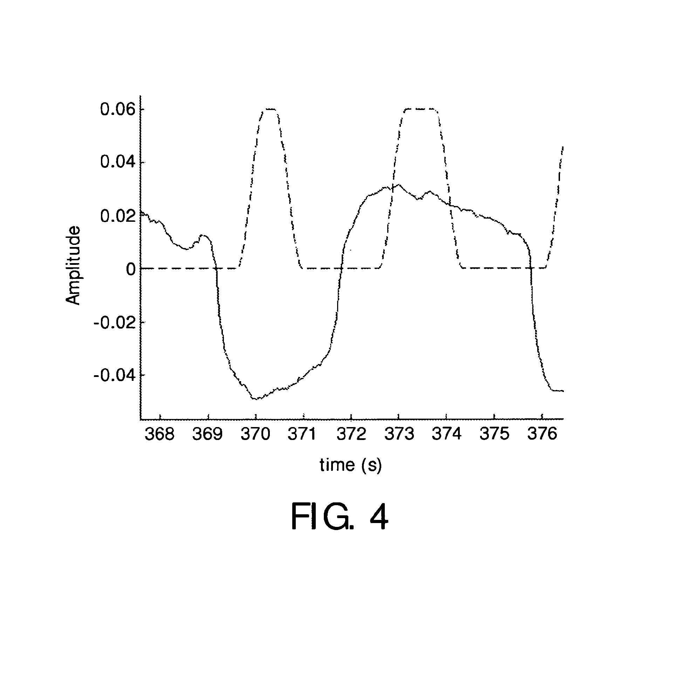

In some implementations, the sliding window FFT can be applied to each complete inhalation or exhalation segment. However, in preferred embodiments, the sliding window FFT is applied to only a part of each inhalation or exhalation segment around the peak air flow (i.e. where the air flow rate is at a local maximum). This preferred embodiment is illustrated in Figure 4, in which the air flow samples are represented by the solid line (negative values again representing inhalation and positive values representing exhalation), and the dashed line indicates the samples to which the sliding window FFT is applied. Thus, it can be seen that the sliding window FFT is applied at and around the samples where the peak air flow occurs during each inhalation and exhalation. It has been found that this narrow sliding window approach provides a better data set for use in subsequent analysis by the processor 12.

In a particular embodiment, the width of the window is less than one second (so for example at a sample rate of 2600 Hz a FFT sliding window of width $2^{11} = 2048$ is used, and for a sample rate of 26000 Hz a FFT sliding window of width $2^{14} = 16384$ is used). The FFT window is then shifted by three-quarters of the FFT window length.

The N frequency-transformed breathing cycles are then averaged to provide separate mean frequency spectrums for inhalation and exhalation.

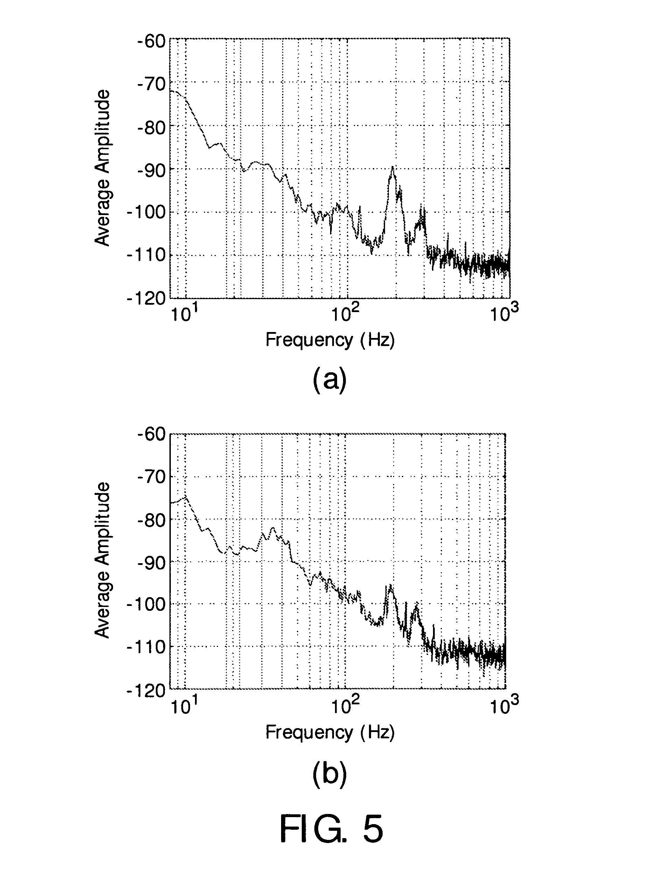

It has been found that the frequency spectrum obtained from air flow sample data for patients with a breathing disorder, such as obstructive sleep apnea, differs from the frequency spectrum obtained from healthy patients. For example, changes have been identified in certain frequency ranges or bands below 100Hz, most notably the 18-22Hz and 30-40Hz frequency bands. These changes are illustrated in Figure 5 which shows the mean exhalation frequency spectrum for a healthy patient (Figure 5(a)) and a patient with obstructive sleep apnea (Figure 5(b)). Thus, it can be seen, for example, that there is an elevation in the 30-40 Hz frequency band and a reduction in the 18-22 Hz frequency band for a patient with OSA compared to a healthy patient. Similar characteristics have been found in the mean inhalation frequency spectrum.

Thus, in accordance with the invention, the processor 12 extracts values for one or more parameters from the frequency spectrum or spectrums determined in the frequency analysis processing step 36. In particular, the value for at least one parameter is determined from the signals in one or more frequency bands covering frequencies that are below 100 Hz.

Various different parameters can be extracted in the feature extraction step 38 according to the invention.

One parameter that can be extracted is the difference between the mean exhalation frequency amplitude in a first frequency band, for example the range of 20-50 Hz, or, more preferably, 25-45 Hz, or, even more preferably, 30-40 Hz (denoted $f_{ex30-40}$), and the mean exhalation frequency amplitude in a second frequency band, for example preferably the range of 12-30 Hz, or, more preferably, 15-25 Hz, or, even more preferably, 18-22 Hz (denoted $f_{ex18-22}$). The parameter value can be given by $f_{ex30-40} - f_{ex18-22}$, and according to the observation described above, the value of the parameter for a healthy patient will generally be negative, whereas the value will generally be higher for a patient with OSA. Thus, the value of this parameter can be used by a physician or the apparatus 2 as an indicator as to whether the patient has OSA (perhaps by comparison to a threshold based on the parameter value(s) obtained for one or more healthy, non-OSA, patients). It will be appreciated by those skilled in the art that a value for a similar parameter can be obtained from the difference between the mean inhalation frequency amplitude in these or similar frequency ranges.

Furthermore, it will also be appreciated by those skilled in the art that the first and second frequency bands described above can be varied from the exemplary values given without substantially affecting the usefulness of the parameter in helping to diagnose OSA. For example, one or both of the most preferable frequency bands described above can be narrower (i.e. covering a smaller range of frequencies, for example 32-38 Hz and 19-21 Hz respectively), wider (i.e. covering a larger range of frequencies, for example 28-42 Hz and 17-23 Hz respectively) and/or shifted along the frequency spectrum (for example 28-38 Hz and 17-21 Hz respectively).

Another parameter that can be extracted is the difference between the mean exhalation frequency amplitude in a third frequency band, for example the range of 0-20 Hz, or, more preferably, 0-15 Hz, or, even more preferably, 0-10 Hz (denoted $f_{ex0-10}$) and the mean inhalation frequency amplitude in the same or a similar frequency band, for example the range 0-20 Hz, or, more preferably, 0-15 Hz, or, even more preferably, 0-10 Hz (denoted $f_{in0-10}$). The parameter value can be given by $f_{ex0-10} - f_{in0-10}$. The value of the parameter will be generally close to zero for a healthy patient, whereas the value will generally be higher for a patient with OSA. Thus, as with the first parameter above, the value of this parameter can be used by a physician or the apparatus 2 as an indicator as to whether the patient has OSA (perhaps by comparison to a threshold based on the parameter value(s) obtained for one or more healthy, non-OSA, patients).

As with the first parameter described above, it will be appreciated by those skilled in the art that the third frequency band described above can be varied from the exemplary value given without substantially affecting the usefulness of the parameter in helping to diagnose OSA. For example, the most preferable frequency band described above can be narrower (i.e. covering a smaller range of frequencies, for example 0-9 Hz), wider (i.e. covering a larger range of frequencies, for example 0-12 Hz) and/or shifted along the frequency spectrum (for example 2-12 Hz).

A further parameter that can be extracted is the difference between the mean frequency amplitude in the range 0-100 Hz for inhalation or exhalation (denoted $f_{in0-100}$ or $f_{ex0-100}$ as appropriate) and a 'noise' level at frequencies above 100 Hz. Again, these frequency bands can be varied from the exemplary value given without substantially affecting the usefulness of the parameter in helping to diagnose OSA (for example the threshold frequency for the noise level could be set higher than 100 Hz, for example 200 Hz or 2000 Hz).

Those skilled in the art will appreciate that the mean exhalation or inhalation frequency amplitude in a particular frequency band can be obtained from the output of the frequency analysis step 36 by averaging the amplitude of the frequency domain signal in the specified frequency band.

It will also be appreciated that the invention is not limited to the extraction of the specific parameters set out above, and that information useful for characterizing the breathing condition of a patient can be obtained from various other parameters that can be readily contemplated by those skilled in the art. In particular, and as discussed above, parameters can be extracted from frequency bands other than those specified above. Furthermore, it is not essential for the parameter or parameters to be based on the mean amplitude in a specified frequency band, since comparable results can be derived using other mathematical operations such as the area under the plot of the frequency spectrum in the frequency band or from the square of the amplitude.

One advantage of using the parameters described above (including the variations to the various frequency bands) is that, by comparing one part of the frequency spectrum for a patient to another part of the spectrum for the same patient, there is no need for the apparatus 2 to be calibrated for each new patient that is to be tested, which reduces the time required for the testing procedure.

In addition to extracting values for one or more parameters from the signals in the frequency domain, the processor 12 can extract values for other parameters from the time domain samples provided by the pneumotachograph 4 (whether the raw data or the data following the preprocessing step 34) during the feature extraction step 38.

For example, the processor 12 can extract time-domain features such as mean breathing cycle length and mean ratio between the length of the inhalation and length of the exhalation

Once the required parameter values have been extracted from the data, the processor 12 can either present the parameter values to a physician or other healthcare professional via the display 14 (or other visual output such as a printer-generated document) for use in assisting the physician to arrive at a diagnosis for the patient, or the processor 12 can perform a further processing step to combine the parameter values into a single useful score value.

In this feature combination step 40, the processor 12 can combine the extracted values of multiple parameters into a single score that can be used to assist in the diagnosis of a breathing disorder, as it has been found that a score based on the value of a number of the parameters described above is more useful in the reliable diagnosis of a breathing disorder than individual parameter values.

In one embodiment, the parameter values are combined linearly, for example:

Score,

$$s = a + b.p_1 + c.p_2 + \ldots n.p_n$$

where $p_1$, $p_2$, ..., $p_n$ denote the extracted values for respective parameters, and a, b, c, ..., n are constant values, although other ways of combining the parameter values are within the scope of the invention, such as non-linear combinations or the use of decision trees.

In further embodiments, the score can also be based on other patient-related parameters, such as body-mass index (BMI), age, sex, Mallampati score, etc. which can be manually input to the apparatus 2 by the patient or operator.

In a particular embodiment, a score $S_{OSA}$ useful in the assessment of a patient that might have OSA is given by

$$s_{OSA} = -3.21 + 0.13 * p_1 + 0.13 * p_2 + 0.14 * p_3$$

where $p_1$ is the patient's BMI, $p_2$ is the difference between the mean amplitude between inhalation and exhalation in the frequency range 0-10 Hz, and $p_3$ is the difference in the mean amplitude in the frequency range 30-40 Hz and 18-22 Hz during exhalation. A positive value for $S_{OSA}$ indicates that the patient is likely to have, or has OSA, whereas a negative value for $S_{OSA}$ indicates that the patient is not likely to have OSA.

After the calculation of the score s, the result is displayed by the apparatus 2 (step 42 of the flow chart in Figure 2). The score can be used by a physician or other healthcare professional to determine whether the patient has OSA or any other breathing disorder.

Alternatively, or in addition, the apparatus 2 can compare the determined score to one or more thresholds to determine an indication of whether the patient has a breathing disorder. In this case, the apparatus 2 can display the score and the indication (and optionally the parameter values used to calculate the score) to the operator of the apparatus 2.

As indicated above, if feature combination step 40 is omitted, the display step 42 can merely comprise displaying the value of the parameter or parameters determined in step 38. The parameter value or values can be noted by a physician or other healthcare professional and used to assist the physician in determining whether the patient has OSA. It will be appreciated that the physician or other healthcare professional can themselves derive a score as described above from the parameter value or values output by the apparatus 2, and optionally compare the score to one or more predetermined thresholds.

It will be appreciated by those skilled in the art that the signal processing method presented above is effectively the analysis of a signal resulting from the turbulence of the air being breathed in and out by the patient, which can be understood as a 'sound' overlying the air flow itself. Therefore, in alternative embodiments of the invention, it is possible for the signal data processed according to the invention to be obtained by a microphone or other sound sensor placed close to the patient (who is awake) while they breathe. These sound measurements can then be processed in a similar way to the air flow rate measurements described above, and the values for the appropriate parameters obtained.

There is therefore provided a method and apparatus for collecting information on a patient that can be used in diagnosing obstructive sleep apnea in the patient, where the information is collected while the patient is awake.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (2) for determining one or more parameters for use in diagnosing the presence of obstructive sleep apnea in a patient while the patient is awake, the apparatus (2) comprising:

   a processor (12) configured to receive signals representing measurements of a patient's breathing obtained during a plurality of breathing cycles by the patient, convert the signals into the frequency domain and to determine a value for at least one parameter based on an analysis of the frequency-domain converted signals in one or more frequency bands covering frequencies below 100 Hz,
   **characterized in that** the processor (12) is configured to determine a value for a first parameter by comparing the signals in a first frequency band during exhalation to the signals in a second frequency band during exhalation, and/or determine a value for a second parameter by comparing the signals in a third frequency band during inhalation to the signals in the third frequency band during exhalation, and/or determine a value for a third parameter by comparing the signals in a fourth frequency band during an inhalation or exhalation to a noise level above a frequency threshold during the inhalation or exhalation.

2. An apparatus (2) as claimed in claim 1, wherein the processor (12) is configured to determine whether the patient is likely to have obstructive sleep apnea based on the value of the at least one parameter and to output an indication of the likely presence or absence of obstructive sleep apnea in the patient to an operator of the apparatus (2).

3. An apparatus (2) as claimed in claim 2, wherein the processor (12) is configured to determine whether the patient is likely to have obstructive sleep apnea based on a combination of values for a plurality of parameters.

4. An apparatus (2) as claimed in claim 1, wherein the processor (12) is configured to determine a value for the first parameter by comparing the signals during exhalation in the frequency band 30-40 Hz to the signals during exhalation in the frequency

5. An apparatus (2) as claimed in claim 1, wherein the processor (12) is configured to determine the value for the second parameter based on the signals in the frequency band 0-10 Hz.

6. An apparatus (2) as claimed in any preceding claim, wherein the processor (12) is configured to receive signals indicative of the rate of air flow during the plurality of breathing cycles by the patient.

7. An apparatus (2) as claimed in claim 6, further comprising:

   an air flow measuring device (4) for measuring the flow rate of air over time during the plurality of breathing cycles by the patient and for generating the signals indicative of the rate of air flow during the breathing cycles.

8. An apparatus (2) as claimed in any of claims 1 to 5, wherein the processor (12) is configured to receive signals indicative of the sound of the patient's breathing during the plurality of breathing cycles by the patient.

9. An apparatus (2) as claimed in any preceding claim, wherein the processor (12) is configured to convert the signals into the frequency domain by identifying the peak air flow during each inhalation and exhalation part of the breathing cycle and performing a Fast Fourier Transform, FFT, on the signals around the peak flows in each inhalation and exhalation part of the breathing cycle.

10. A method for determining one or more parameters for use in diagnosing obstructive sleep apnea, comprising:

    obtaining signals representing measurements of a patient's breathing during a plurality of breathing cycles by the patient while the patient is awake (32);
    converting the signals into the frequency domain (36);
    the method being **characterized in** further comprising
    determining a value for a first parameter by comparing the signals in a first frequency band during exhalation to the signals in a second frequency band during exhalation; and/or
    determining a value for a second parameter by comparing the signals in a third frequency band during inhalation to the signals in the third frequency band during exhalation; and/or
    determining a value for a third parameter by comparing the signals in a fourth frequency band during an inhalation or exhalation to a noise level above a frequency threshold during the inhalation or exhalation;
    wherein each of the first, second and third frequency bands covers frequencies below 100 Hz (38).

11. A method as claimed in claim 10, wherein the first frequency band is 30-40 Hz, the second frequency band is 18-22 Hz, the third frequency band is 0-10 Hz, the fourth frequency band is 0-100Hz and the frequency threshold is 100 Hz or above.

**Patentansprüche**

1. Gerät (2) zum Ermitteln von einem oder mehreren Parametern zur Verwendung für der Diagnose des Vorliegens von obstruktiver Schlafapnoe bei einem Patienten, während der Patient wach ist, wobei das Gerät (2) Folgendes umfasst:

   einen Prozessor (12), der eingerichtet ist, um Signale zu empfangen, die Messungen der Atmung eines Patienten darstellen, welche während einer Vielzahl von durch den Patienten durchgeführten Atemzyklen erlangt wurden, die Signale in den Frequenzbereich umzuwandeln und einen Wert für mindestens einen Parameter basierend auf einer Analyse der in den Frequenzbereich umgewandelten Signale in einem oder mehreren Frequenzbändern, die Frequenzen unterhalb von 100 Hz abdecken, zu ermitteln;
   **dadurch gekennzeichnet, dass** der Prozessor (12) eingerichtet ist, um einen Wert für einen ersten Parameter zu ermitteln, indem er die Signale in einem ersten Frequenzband während des Ausatmens mit Signalen in einem zweiten Frequenzband während des Ausatmens vergleicht, und/oder um einen Wert für einen zweiten Parameter zu ermitteln, indem er die Signale in einem dritten Frequenzband während des Einatmens mit den Signalen in dem dritten Frequenzband während des Ausatmens vergleicht, und/oder um einen Wert für einen dritten Parameter zu ermitteln, indem er die Signale in einem vierten Frequenzband während eines Einatmens oder Ausatmens mit einem Rauschpegel über einem Frequenzschwellenwert während des Einatmens oder Ausatmens vergleicht.

2. Gerät (2) nach Anspruch 1, wobei der Prozessor (12) eingerichtet ist, um basierend auf dem Wert von dem mindestens einen Parameter zu ermitteln, ob der Patient wahrscheinlich eine obstruktive Schlafapnoe hat, und um eine Angabe bezüglich des wahrscheinlichen Vorliegens oder der Abwesenheit von obstruktiver Schlafapnoe bei dem Patienten an einen Bediener des Geräts (2) auszugeben.

3. Gerät (2) nach Anspruch 2, wobei der Prozessor (12) eingerichtet ist, um basierend auf einer Kombination von Werten für eine Vielzahl von Parametern zu ermitteln, ob der Patient wahrscheinlich eine obstruktive Schlafapnoe hat.

4. Gerät (2) nach Anspruch 1, wobei der Prozessor (12) eingerichtet ist, um einen Wert für den ersten Parameter zu ermitteln, indem er die Signale während des Ausatmens in dem Frequenzband 30-40 Hz mit Signalen während des Ausatmens in dem Frequenzband 18-22 Hz vergleicht.

5. Gerät (2) nach Anspruch 1, wobei der Prozessor (12) eingerichtet ist, um den Wert für den zweiten Parameter basierend auf den Signalen in dem Frequenzband 0-10 Hz zu ermitteln.

6. Gerät (2) nach einem der vorhergehenden Ansprüche, wobei der Prozessor (12) eingerichtet ist, um Signale zu empfangen, die die Luftströmungsrate während der Vielzahl von durch den Patienten durchgeführten Atemzyklen angeben.

7. Gerät (2) nach Anspruch 6, weiterhin Folgendes umfassend:

eine Luftströmungsmessvorrichtung (4) zum Messen der Strömungsrate der Luft über die Zeit während der Vielzahl von durch den Patienten durchgeführten Atemzyklen und zum Erzeugen der Signale, die die Luftströmungsrate während der Atemzyklen angeben.

8. Gerät (2) nach einem der Ansprüche 1 bis 5, wobei der Prozessor (12) eingerichtet ist, um Signale zu empfangen, die das Geräusch des Atmens durch Patienten während der Vielzahl der durch den Patienten durchgeführten Atemzyklen angeben.

9. Gerät (2) nach einem der vorhergehenden Ansprüche, wobei der Prozessor (12) eingerichtet ist, um die Signale in den Frequenzbereich umzuwandeln, indem er die Spitzenluftströmung während jedes Einatmungs- und Ausatmungsteils des Atemzyklus identifiziert und eine Fast Fourier Transformation, FFT, für die Signale um die Spitzenströmungen herum in jedem Einatmungs- und Ausatmungsteil des Atemzyklus durchführt.

10. Verfahren zum Ermitteln von einem oder mehreren Parametern zur Verwendung für die Diagnose von obstruktiver Schlafapnoe, wobei das Verfahren Folgendes umfasst:

Erlangen von Signalen, die in wachem Zustand des Patienten durchgeführte Messungen der Atmung eines Patienten während einer Vielzahl von durch den Patienten durchgeführten Atemzyklen darstellen (32);
Umwandeln der Signale in den Frequenzbereich (36);
wobei das Verfahren **dadurch gekennzeichnet ist, dass** es weiterhin Folgendes umfasst:
Ermitteln eines Werts für einen ersten Parameter durch Vergleichen der Signale in einem ersten Frequenzband während des Ausatmens mit den Signalen in einem zweiten Frequenzband während des Ausatmens; und/oder
Ermitteln eines Werts für einen zweiten Parameter durch Vergleichen der Signale in einem dritten Frequenzband während des Einatmens mit den Signalen in dem dritten Frequenzband während des Ausatmens; und/oder
Ermitteln eines Werts für einen dritten Parameter durch Vergleichen der Signale in einem vierten Frequenzband während eines Einatmens oder Ausatmens mit einem Rauschpegel über einem Frequenzschwellenwert während des Einatmens oder Ausatmens;
wobei jedes der Frequenzbänder, das erste, zweite und dritte, Frequenzen unter 100 Hz abdeckt (38).

11. Verfahren nach Anspruch 10, wobei das erste Frequenzband 30-40 Hz beträgt, das zweite Frequenzband 18-22 Hz beträgt, das dritte Frequenzband 0-10 Hz beträgt, das vierte Frequenzband 0-100 Hz beträgt und der Frequenzschwellenwert 100 Hz oder mehr beträgt.

**Revendications**

1. Appareil (2) pour déterminer un ou plusieurs paramètres pour l'utilisation dans le diagnostic de la présence d'apnée du sommeil obstructive chez un patient alors que le patient est éveillé, l'appareil (2) comprenant :

   un processeur (12) configuré pour recevoir des signaux représentant des mesures de la respiration d'un patient obtenues durant une pluralité de cycles de respiration par le patient, convertir les signaux en le domaine de fréquence et pour déterminer une valeur pour au moins un paramètre en fonction d'une analyse des signaux convertis en domaine de fréquence dans une ou plusieurs bandes de fréquence couvrant des fréquences inférieures à 100 Hz,
   **caractérisé en ce que** le processeur (12) est configuré pour déterminer une valeur pour un premier paramètre en comparant les signaux dans une première bande de fréquence durant l'exhalaison aux signaux dans une deuxième bande de fréquence durant l'exhalaison, et/ou déterminer une valeur pour un deuxième paramètre en comparant les signaux dans une troisième bande de fréquence durant l'inhalation aux signaux dans la troisième bande de fréquence durant l'exhalaison, et/ou déterminer une valeur pour un troisième paramètre en comparant les signaux dans une quatrième bande de fréquence durant une inhalation ou exhalaison à un niveau de bruit supérieur à un seuil de fréquence durant l'inhalation ou l'exhalaison.

2. Appareil (2) selon la revendication 1, dans lequel le processeur (12) est configuré pour déterminer si le patient souffre vraisemblablement d'apnée du sommeil obstructive en fonction de la valeur de l'au moins un paramètre et pour fournir une indication de la présence ou de l'absence vraisemblable d'apnée du sommeil obstructive chez le patient à un opérateur de l'appareil (2).

3. Appareil (2) selon la revendication 2, dans lequel le processeur (12) est configuré pour déterminer si le patient souffre vraisemblablement d'apnée du sommeil obstructive en fonction d'une combinaison de valeurs pour une pluralité de paramètres.

4. Appareil (2) selon la revendication 1, dans lequel le processeur (12) est configuré pour déterminer une valeur pour le premier paramètre en comparant les signaux durant l'exhalaison dans la bande de fréquence 30 à 40 Hz aux signaux durant l'exhalaison dans la bande de fréquence 18 à 22 Hz.

5. Appareil (2) selon la revendication 1, dans lequel le processeur (12) est configuré pour déterminer la valeur pour le deuxième paramètre en fonction des signaux dans la bande de fréquence 0 à 10 Hz.

6. Appareil (2) selon une quelconque revendication précédente, dans lequel le processeur (12) est configuré pour recevoir des signaux indicatifs de la vitesse d'écoulement d'air durant la pluralité de cycles de respiration par le patient.

7. Appareil (2) selon la revendication 6, comprenant en outre : un dispositif de mesure d'écoulement d'air (4) pour mesurer la vitesse d'écoulement d'air au fil du temps durant la pluralité de cycles de respiration par le patient et pour générer les signaux indicatifs de la vitesse d'écoulement d'air durant les cycles de respiration.

8. Appareil (2) selon l'une quelconque des revendications 1 à 5, dans lequel le processeur (12) est configuré pour recevoir des signaux indicatifs du son de la respiration du patient durant la pluralité de cycles de respiration par le patient.

9. Appareil (2) selon une quelconque revendication précédente, dans lequel le processeur(12) est configuré pour convertir les signaux en le domaine de fréquence en identifiant l'écoulement d'air de pointe durant chaque partie d'inhalation et d'exhalaison du cycle de respiration et réaliser une transformée de Fourier rapide, FFT, sur les signaux autour des écoulements de pointe dans chaque partie d'inhalation et d'exhalaison du cycle de respiration.

10. Procédé pour déterminer un ou plusieurs paramètres pour l'utilisation dans le diagnostic d'apnée du sommeil obstructive, comprenant :

    l'obtention de signaux représentant des mesures de respiration d'un patient durant une pluralité de cycles de respiration par le patient alors que le patient est éveillé (32) ;
    la conversion des signaux en le domaine de fréquence (36) ; le procédé étant **caractérisé en ce qu'**il comprend en outre :

la détermination d'une valeur pour un premier paramètre en comparant les signaux dans une première bande de fréquence durant l'exhalaison aux signaux dans une deuxième bande de fréquence durant l'exhalaison ; et/ou

la détermination d'une valeur pour un deuxième paramètre en comparant les signaux dans une troisième bande de fréquence durant l'inhalation aux signaux dans la troisième bande de fréquence durant l'exhalaison ; et/ou

la détermination d'une valeur pour un troisième paramètre en comparant les signaux dans une quatrième bande de fréquence durant une inhalation ou exhalaison à un niveau de bruit supérieur à un seuil de fréquence durant l'inhalation ou l'exhalaison ;

dans lequel chacune des première, deuxième et troisième bandes de fréquence couvre des fréquences inférieures à 100 Hz (38).

11. Procédé selon la revendication 10, dans lequel la première bande de fréquence est 30 à 40Hz, la deuxième bande de fréquence est 18 à 22 Hz, la troisième bande de fréquence est 0 à 10 Hz, la quatrième bande de fréquence est 0 à 100Hz et le seuil de fréquence est 100 Hz ou plus.

FIG. 1

FIG. 2

(a)

(b)

FIG. 3

EP 2 621 336 B1

FIG. 4

(a)

(b)

FIG. 5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6942626 B

- US 2007093724 A

**Non-patent literature cited in the description**

- Investigation of Obstructive Sleep Apnea Using Nonlinear Mode Interactions in Nonstationary Snore Signals. **NG et al.** Annals of Biomedical Engineering. September 2009, vol. 37, 1796-1806